(19) Europäisches Patentamt
European Patent Office
Office européen des brevets

(11) Numéro de publication : **0 184 243**
**B1**

(12) # FASCICULE DE BREVET EUROPÉEN

(45) Date de publication du fascicule du brevet : **01.02.89**

(21) Numéro de dépôt : **85201850.6**

(22) Date de dépôt : **12.11.85**

(51) Int. Cl.⁴ : **C 07 K 1/00**, C 07 K 1/08, A 61 K 37/02// C07K5/00, C07K7/00

(54) **Procédé pour la synthèse de peptides.**

**Deux requêtes en rectification de la description ont été présentées conformément à la règle 88 CBE.**

(30) Priorité : **23.11.84 FR 8418011**

(43) Date de publication de la demande : **11.06.86 Bulletin 86/24**

(45) Mention de la délivrance du brevet : **01.02.89 Bulletin 89/05**

(84) Etats contractants désignés : **AT BE CH DE FR GB IT LI LU NL SE**

(56) Documents cités :
**EP–A– 0 084 941**
**EP–A– 0 099 709**
**FR–A– 2 341 586**
**CHEMICAL ABSTRACTS, vol. 100, no. 12, 1984, page 14, réf. no. 86324b, Columbus, Ohio, US; & US - A - 4 419 299 (DOW CHEMICAL CO.) 06.12.1983**
**AGRICULTURAL AND BIOLOGICAL CHEMISTRY, vol. 39, no. 2, 1975, pages 571-572, The Agricultural Chemical Society of Japan; YOSUKE NAKAJIMA et al.: "Novel synthesis of alpha-amino acids via cyanosilylation of schiff bases"**
**JOURNAL OF MEDICINAL CHEMISTRY, vol. 25, no. 5, mai 1982, pages 605-610, American Chemical Society; R.L. JOHNSON: "Renin inhibitors. Substitution of the leucyl residues of leu-leu-val-phe-OCH3 with 3-amino-2-hydroxy-5-methylhexanoic acid"**
**Le dossier contient des informations techniques présentées postérieurement au dépôt de la demande et ne figurant pas dans le présent fascicule.**

(73) Titulaire : **SOLVAY & Cie (Société Anonyme)**
**Rue du Prince Albert, 33**
**B-1050 Bruxelles (BE)**

(72) Inventeur : **Anteunis, Marc**
**De Campagne 5**
**B-9910 Gand (Mariakerke) (BE)**
Inventeur : **Becu, Christian**
**Boonstede 44**
**B-9810 Gand (Baarle-Drongen) (BE)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**EP 0 184 243 B1**

Jouve, 18, rue St-Denis, 75001 Paris, France

**Description**

La présente invention concerne un procédé pour la synthèse de peptides à partir d'acides aminés, et à l'intervention de trialkylcyanosilanes.

La silylation des acides aminés pour préparer des peptides par réaction d'un acide aminé, dont la fonction $—NH_2$ est bloquée et dont la fonction —COOH est activée, avec un autre acide aminé, silylé à l'intervention d'agents de silylation tels que le triméthylchlorosilane, a été décrite par Kricheldorf H.R., Liebigs Ann., 1972, 763, p. 17-38.

Par ailleurs, d'autres agents de silylation tels que l'hexaméthylsilazane (Birkofer L., Konkol W. et Ritter A., Chem. Ber. 1961, 94, p. 1263-1267 et Birkofer L., Ritter A. et Neuhausen P., Liebigs Ann. Chem., 1962, p. 190-199) et la N-(triméthylsilyl)-diéthylamine et la N,O-bis(triméthylsilyl)acétamide (S.V. Rogozhin, Yu. A. Davidovich, A.I. Yurtanov, Seriyta Khimicheskaya, N. 3, p. 657-660, March 1977 — Original article October 27, 1976) ont également été divulgués pour cette même réaction.

On a également proposé (brevet FR-A-2 341 586) de mettre en œuvre un groupe bêta-silyléthyle de formule

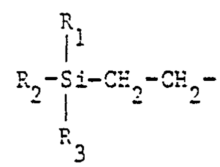

dans laquelle $R_1$, $R_2$ et $R_3$ représentent un radical hydrocarboné, pour la protection des groupes carboxyle lors de la synthèse de séquences de peptides.

Les divers agents de silylation utilisés à ce jour présentent toutefois des inconvénients divers. En effet, ces agents nécessitent en général la présence d'une base qui doit être éliminée par la suite et qui provoque souvent la racémisation des acides aminés lors de l'étape de couplage ou la cyclisation interne au stade dipeptide. En outre des produits secondaires sont formés lors de la silylation et la réaction est généralement incomplète ce qui engendre des rendements faibles souvent inférieurs à 50 %.

Un autre inconvénient des agents de silylation de l'art antérieur est dû à la présence d'eau qui décompose les réactifs alkylsilylés avec formation de l'hexaalkyldisiloxane correspondant.

Par ailleurs, les dérivés silylés sont formés au cours d'une étape séparée et doivent être isolés avant l'étape de couplage.

Dans les procédés de l'art antérieur, la synthèse des peptides est en outre fortement limitée par les problèmes de solubilité des peptides formés lorsque leur poids moléculaire augmente. La synthèse est donc souvent limitée par les faibles solubilités des produits intermédiaires durant les réactions de couplage et par les difficultés de purification des produits finaux. Plus la chaîne peptidique est longue, plus les problèmes sont complexes.

Par ailleurs, certains agents de silylation particuliers de l'art antérieur présentent des problèmes propres. C'est ainsi que

— l'emploi d'hexaméthyldisilazane, réactif plus nucléophile que le composé aminé silylé désiré, provoque des réactions secondaires qui affectent le rendement du procédé.

— l'utilisation du triméthylchlorosilane est incompatible avec la protection des groupements $—NH_2$ des acides aminés par des groupements de type benzyloxycarbonyle.

D'autre part, l'emploi de triméthylcyanosilane a été proposé comme agent de réaction pour des aldéhydes (Journal of Medicinal Chemistry, vol. 25, nº 5, mai 1982, pages 605-610) ou des cétones, ainsi que comme agent protecteur pour des groupes carbonyles (Agricultural and Biological Chemistry, vol. 39, nº 2, 1975, pages 571-572).

Le procédé de la présente invention vise à réaliser la synthèse de peptides à partir d'acides aminés par des agents de silylation ne présentant pas les inconvénients des procédés connus.

Plus particulièrement, le procédé permet une réaction de couplage rapide en continu, qui a lieu sans racémisation, pouvant être réalisé en l'absence de coréactifs basiques, en présence éventuelle d'eau et en présence des agents de protection connus. En outre, il permet l'obtention de peptides de poids moléculaire élevé avec des rendements supérieurs à ceux obtenus avec les agents de silylation connus. Le procédé de l'invention permet par ailleurs de consommer chimiquement l'eau et d'obtenir des dérivés silylés volatifs ce qui facilite leur élimination.

A cet effet, la présente invention concerne un procédé pour la synthèse de peptides à partir

a) d'acides aminés éventuellement associés et/ou substitués, qui sont activés, et

b) d'acides aminés éventuellement associés et/ou substitués, qui sont silylés à l'intervention de dérivés trialkylsilanes dans lequel on utilise comme trialkylsilanes des trialkylcyanosilanes de formule générale (A)

$$R_2 - \underset{\substack{| \\ R_3}}{\overset{\substack{R_1 \\ |}}{Si}} - C \equiv N \qquad (A)$$

dans laquelle $R_1$, $R_2$, $R_3$ représentent, indépendamment les uns des autres des groupements alkyles qui peuvent être identiques ou différents et qui contiennent de 1 à 3 atomes de carbone. Habituellement, les groupements $R_1$, $R_2$, et $R_3$ représentent des groupements alkyles contenant de 1 à 2 atomes de carbone. Enfin, $R_1$, $R_2$ et $R_3$ représentent de préférence des groupements alkyles identiques. La triméthylcyanosilane est tout particulièrement préféré.

Les trialkylcyanosilanes utilisés dans le procédé selon l'invention sont de préférence des produits volatils qui peuvent être facilement éliminés. Ces agents de silylation possèdent des propriétés de solubilisation supérieure aux agents de silylation habituels ce qui permet leur utilisation simultanée comme réactif pour la réalisation de la liaison peptidique entre les acides aminés et comme solvant des acides aminés et des peptides.

Les trialkylcyanosilanes peuvent être employés seuls, ou en présence de tiers solvants, ces derniers pouvant par exemple provenir de l'étape d'activation ou être ajoutés conjointement lors de l'étape de couplage. On préfère toutefois réaliser l'étape de couplage sans addition de tiers solvants.

Des tiers solvants qui ont donné de bons résultats lorsqu'ils sont utilisés conjointement au triméthylcyanosilane sont notamment le dichlorométhane et le tétrahydrofurane.

La quantité de trialkylcyanosilanes que l'on utilise dans le procédé selon l'invention peut varier dans de larges limites. En général, on met en œuvre de 20 à 0,01 ml de trialkylcyanosilane par m.mole d'acide aminé. Dans le cas du triméthylcyanosilane, on met en œuvre de préférence de 5 à 0,1 ml de triméthylcyanosilane par m. mole d'acide aminé mis en œuvre.

Comme acides aminés, on peut utiliser tout acide aminé associé et/ou substitué possédant au moins une fonction carboxylique et au moins une fonction amine primaire ou secondaire, tel que les acides aminés naturels connus ou les acides aminés synthétiques ne se trouvant pas dans la nature. Comme acides aminés naturels, on utilise généralement des acides aminés aliphatiques linéaires, ramifiés ou cycliques, tels que les acides aminés à chaîne hydrocarbonée, les acides aminés hydroxylés ou soufrés, les acides aminés dicarboxyliques, les acides aminés basiques ainsi que les acides aminés aromatiques ou hétérocycliques.

Par acide aminé associé on entend tout composé résultant de la réaction d'un acide aminé, par au moins une de ses fonctions carboxyliques et/ou fonctions amines, tel que défini ci-avant, avec une entité avec laquelle cette fonction est réactive. Parmi les acides aminés associés préférés on entend des molécules tels que de petits peptides comportant une succession de 2,3 ou plusieurs acides aminés de natures chimiques identiques ou différentes.

Par acide aminé substitué on entend tout composé du type des acides aminés ou acides aminés associés tels que définis ci-dessus comportant en lieu et place d'un ou de plusieurs atomes d'hydrogène, liés à des atomes de carbone, des substituants organiques ou inorganiques. Ces substituants organiques ou inorganiques peuvent être simples ou complexes, substitués ou non, et comprennent notamment, les atomes de chlore et de fluor ainsi que des groupements aliphatiques et aromatiques tels que des groupements alkyls, alkényls, cycloalkyls, benzyls, phényls, napthyls, pyridinyls, etc.

Les autres conditions opératoires utilisées dans le procédé selon l'invention ne sont pas critiques ; ainsi la pression à laquelle est effectué le procédé est généralement comprise entre 0,1 et 10 bar. De bons résultats ont été obtenus à pression atmosphérique. La température à laquelle est effectué le procédé est telle que l'agent de silylation utilisé reste liquide à la pression considérée. Habituellement, elle est comprise entre 0 et 100 °C. A pression atmosphérique lorsqu'on emploie le triméthylcyanosilane comme agent de silylation, la température est de préférence comprise entre 10 et 40 °C lors de la réaction de couplage et de bons résultats ont été obtenus à la température ambiante.

Le procédé peut être réalisé dans tout appareillage conçu à cet effet.

Le schéma de synthèse du procédé selon la présente invention est repris ci-après.

Dans ce schéma $A_1$, $A'_1$, $A_2$ représentent des restes d'acides aminés quelconques naturels ou synthétiques. Il est à noter que le principe du schéma, bien qu'établi pour des acides aminés à fonction amine primaire, s'applique également dans le cas des acides aminés comportant une fonction amine secondaire tels que la proline et l'hydroxyproline ; dans ce cas, il suffit de substituer la fonction $—NH_2$ par une fonction >NH dans le schéma ci-après.

Schéma de synthèse

1 — protection de la fonction amine d'un premier acide aminé par un agent de protection classique, comme par exemple la protection par des groupements de type benzyloxycarbonyle (appelé ci-après Z) ou tert-butyloxycarbonyle (t-Boc), selon les schémas :

acide aminé ⟶ acide aminé protégé

$$NH_2—A_1—COOH \xrightarrow{Z} Z—NH—A_1—COOH$$

$$NH_2—A_1—COOH \xrightarrow{t\text{-}Boc} t\text{-}Boc—NH—A_1—COOH$$

2 — activation de la fonction carboxyle par un agent d'activation classique, comme par exemple l'activation par la transformation en chlorure d'acide ou anhydride (Act) selon les schémas :

acide aminé protégé → acide aminé protégé et activé

$$Z—NH—A_1—COOH \rightarrow Z—NH—A_1—COOAct \quad (I)$$
$$t\text{-}Boc—NH—A_1—COOH \rightarrow t\text{-}Boc—NH—A_1—COOAct \quad (I)'$$

Il est toutefois à remarquer que dans certains cas il est possible de réaliser l'activation à partir d'acides aminés protégés et dont la fonction carboxylique est substituée. Un exemple de ce type sont les acides aminés N- protégés silylés obtenus par silylation préalable par le triméthylcyanosilane (TMSCN), selon le schéma :

$$t\text{-}Boc—NH—A_1—COOTMS \rightarrow t\text{-}Boc—NH—A_1—COOAct \quad (I)''$$

3 — silylation d'un second acide aminé par réaction avec un trialkylcyanosilane tel que le triméthylcyanosilane (TMSCN) selon le schéma :

acide aminé → acide aminé silylé

$$NH_2—A_2—COOH+TMSCN \rightarrow TMS—NH—A_2—COOTMS+HCN \quad (II)$$

Il est toutefois bien entendu que la distinction entre les étapes 1, 2 et 3 est purement formelle puisque ces étapes peuvent être menées indépendamment les unes des autres. Dès lors, l'étape 3, par exemple, peut indifféremment suivre ou précéder les étapes 1 et 2.

4 — couplage avec formation d'un peptide par réaction de l'acide aminé protégé et activé (I) ou (I)' et de l'acide aminé silylé (II) selon les schémas

$$Z—NH—A_1—COOAct + TMS—NH—A_2—COOTMS \rightarrow Z—NH—A_1—CO—NH—A_2—COOTMS + TMSAct \quad (III)$$

ou

$$t\text{-}Boc—NH—A_1—COOAct + TMS—NH—A_2—COOTMS \rightarrow t\text{-}Boc—NH—A_1—CO—NH—A_2—COOTMS + TMSAct \quad (III)'$$

5 — désilylation du peptide obtenu (III) ou (III)' par déplacement du groupement trialkylsilane et formation du groupement acide carboxylique (—COOH), comme par exemple par un traitement en milieu méthanolique ou en présence d'eau. Cette réaction se fait selon les schémas :

$$Z—NH—A_1—CO—NH—A_2—COOTMS \rightarrow Z—NH—A_1—CO—NH—A_2—COOH \quad (IV)$$

ou

$$t\text{-}Boc—NH—A_1—CO—NH—A_2—COOTMS \rightarrow t\text{-}Boc—NH—A_1—CO—NH—A_2—COOH \quad (IV)'$$

Les étapes 5 et 6 peuvent être réalisées en même temps ou successivement.

6 — déprotection du peptide obtenu (IV) ou (IV)'. Cette déprotection peut être réalisée par le déplacement de l'agent de protection et formation du groupement amine ($NH_2$) par toute méthode connue, telle que par exemple un barbotage d'acide chlorhydrique gazeux à travers une solution de dichlorométhane et/ou de tétrahydrofurane, ou par l'acide trifluoroacétique dans du dichlorométhane suivi du passage des peptides sous forme de sels sur une colonne échangeuse d'ions acides. Cette opération peut être illustrée par les schémas généraux suivants :

$$Z—NH—A_1—CO—NH—A_2—COOH \xrightarrow{H^-} NH_2—A_1—CO—NH—A_2—COOH \quad (V)$$

ou

$$t\text{-Boc—NH—}A_1\text{—CO—NH—}A_2\text{—COOH} \longrightarrow NH_2\text{—}A_1\text{—CO—NH—}A_2\text{—COOH} \qquad (V)'$$

Lorsque l'on désire procéder à des couplages successifs d'acides aminés, les étapes 5 et/ou 6 ne sont pas toujours nécessaires. Dans ce cas, deux voies sont possibles, c'est-à-dire en débutant à partir de la terminaison carboxyle et en allant vers la terminaison amine, soit en débutant à partir de la terminaison amine et en allant vers la terminaison carboxyle.

Selon la première voie, le produit (V) est silylé selon le schéma :

$$NH_2\text{—}A_1\text{—CO—NH—}A_2\text{—COOH + TMSCN} \longrightarrow \text{TMS—NH—}A_1\text{—CO—NH—}A_2\text{—COOTMS} \qquad (VI)$$

et ce produit (VI) est ensuite associé avec un acide aminé protégé et activé du type (I) et (I)'.

Selon la seconde voie, le produit obtenu (V) est protégé et activé selon les schémas décrits dans les étapes 1 et 2 ci-dessus et le produit obtenu est mis à réagir avec un nouvel acide aminé triméthysilylé sur les fonctions amine et carboxyle de formule (II).

Un exemple de schéma de ce deuxième cas peut être le suivant

$$t\text{-Boc—NH—}A_1\text{—CONH—}A'_1\text{—COOH} \xrightarrow{\text{Activation}} t\text{-Boc—NH—}A_1\text{—CONH—}A'_1\text{—COOAct}$$

$$t\text{-Boc—NH—}A_1\text{—CONH—}A'_1\text{—COOAct+TMSNH—}A_2\text{—COOTMS} \xrightarrow{\text{TMSCN en excès}} t\text{-Boc—NH—}A_1\text{—CONH—}A'_1\text{—CONH—}A_2\text{—COOTMS}$$

Les procédés schématisés ci-avant permettent grâce à l'emploi de trialkylcyanosilanes de formule générale (A) d'obtenir des peptides, de type naturel ou synthétique n'ayant pas leur équivalent dans la nature.

Ces peptides connus ou nouveaux obtenus selon le procédé de l'invention peuvent être utilisés dans diverses applications telles que notamment la catalyse enzymatique, comme constituants nutritifs ou encore en tant que produits pharmaceutiques à usage vétérinaire ou humain.

Les exemples suivants servent à illustrer l'invention. Dans ces exemples les abréviations suivantes ont été utilisées :

| | |
|---|---|
| Ala | alanine |
| Asp | acide aspartique |
| Cys | cystéine |
| Gly | glycine |
| Met | méthionine |
| Phe | phénylalanine |
| Pro | proline |
| Ser | sérine |
| Thr | thréonine |
| Thz | gamma-thiaproline |
| Tyr | tyrosine |
| Val | valine |
| O Piv | pivaloyloxy(triméthyl acétyloxy |
| O Succ | N-hydroxysuccinimide |

Exemple 1 : couplage d'un peptide avec un acide aminé activé et un acide aminé silylé

1.a : glycine-cystéine

A 1,1 m. mol de cystéine hydratée lyophilisée est ajoutée 0,5 ml de triméthylcyanosilane dans un tube à essai à paroi épaisse et muni d'un bouchon. Le mélange est passé aux ultrasons (sonificateur) jusqu'à l'obtention d'une solution claire. On chauffe le mélange entre 60 et 80 °C pendant 5 minutes jusqu'à dissolution de l'acide aminé.

Ensuite 1 m. mol de glycine, protégée par Z (groupe benzyloxycarbonyle) est activée par O Succ en présence de N,N'-dicyclohexylcarbodiimide, obtenue dans une étape préalable, est ajoutée.

Le couplage entre la cystéine silylée et la glycine protégée et activée est accéléré par un chauffage doux à 40 °C et est terminé après 1 heure à température ambiante.

La réaction est arrêtée par l'addition de 10 ml d'eau. La solution est ensuite lyophilisée et le lyophilisat est mélangé avec du n-hexane.

Après une addition de 10 ml d'eau pour remettre le produit en suspension, celui-ci est centrifugé et le précipité recueilli est séché.

Le rendement de formation en peptide protégé est pratiquement quantitatif (Z-Gly-CysOH) sans contamination d'épimères (Résonance Magnétique Nucléaire (RMN) < 5 %) l'agent de protection Z est finalement libéré par un barbotage d'acide chlorhydrique gazeux dans du dichlorométhane contenant le peptide protégé avec la formation du dipeptide Gly-CysOH · HCl (chlorhydrate du dipeptide).

1.b : Glycine-Glycine
La formation de Z-Gly-GlyOH est obtenue par le même procédé que celui décrit dans l'exemple 1.a, mais en mettant en œuvre 1,1 m.mol de glycine hydratée lyophilisée en lieu et place de la cystéine.

1.c : Alanine-Sérine
La formation de Z-Ala-SerOH est obtenue par le même procédé que celui décrit dans l'exemple 1.a, mais en mettant en œuvre 1,1 m.mol de sérine hydratée lyophilisée en lieu et place de la cystéine et 1 m.mol d'alanine protégée par Z et activée en lieu et place de la glycine.

1.d : Phénylalanine-Sérine
La formation de t-Boc-Phe-SerOH est obtenue par le même procédé, mais en mettant en œuvre 1,1 m.mol de sérine hydratée lyophilisée en lieu et place de la cystéine et 1 m.mol de phénylalanine protégée par t-Boc et activée en lieu et place de la glycine.

Le groupe protégé par t-Boc est libéré par un traitement avec l'acide trifluoroacétique suivi d'une évaporation.

1.e : Proline-Phénylalanine
La formation de t-Boc-Pro-PheOH est obtenue par le même procédé mais en mettant en œuvre 1,1 m.mol de phénylalanine hydratée lyophilisée en lieu et place de la cystéine et 1 m.mol de proline protégée par t-Boc et activée en lieu et place de la glycine.

Exemple 2 : Condensation répétée sans purification des peptides intermédiaires

Préparation de Z-Gly-Phe-SerOH
1,1 m. mol (116 mg) de sérine hydratée en poudre est dissous dans 1 ml de triméthylcyanosilane par un chauffage entre 60 et 80 °C durant moins de 5 minutes.

A ceci, une solution de 1 m. mol (362 mg) de t-Boc-PheOSucc (phénylalanine protégée par le groupement t-Boc et activée par la N-hydroxysuccinimide) dans 10 ml de dichlorométhane ou de tétrahydrofurane est ajoutée.

Après 30 minutes, l'ester actif a disparu.

Le mélange est versé à travers un verre fritté chargé avec 5 g de silicagel, préalablement humidifié avec 1 ml d'eau et lavé avec 2 fois 20 ml de dichlorométhane.

Un barbotage d'acide chlorhydrique est réalisé durant 30 secondes à travers le filtrat qui est ensuite évaporé.

Le sel de dipeptide recueilli est redissous dans 2 ml de triméthylcyanosilane à température ambiante.

On ajoute ensuite 0,98 mol (275 mg) de Z-GlyOSucc (glycine protégée par le groupement Z et activée par la N-hydroxysuccinimide) dans 10 ml de dichlorométhane.

Après 30 minutes à température ambiante la réaction est arrêtée par l'addition de 10 ml d'eau, le précipité recueilli est séché.

Le rendement en Z-Gly-Phe-SerOH est de 88 % (calculé sur Z-GlyOSucc) (350 mg) ; le produit est pur selon tous les critères de RMN.

Exemple 3 : Préparation de Pro-Phe-Met-AspOH avec identification des oligopeptides intermédiaires

1ère étape : t-Boc-Met-AspOH
150 mg (1,1 m.mol) d'acide aspartique hydraté est dissous dans 0,5 ml de triméthylcyanosilane à 80 °C en 3 minutes.

346 mg (1,0 m.mol) t-Boc-MetOSucc est ajouté et le tout est chauffé à 40 °C.

Après une heure, un mélange de 250 mg d'eau dans 2 ml d'acétonitrile est ajouté. Le produit est filtré sur 5 g de $SiO_2$ comme dans l'exemple 2 ci-dessus et le produit obtenu est élué avec 30 ml d'une solution contenant 90 % d'acétonitrile et 10 % de méthanol.

Le résidu est évaporé.

Il contient en quantité équivalente le N-hydroxysuccinimide et le dipeptide désiré protégé par le groupe t-Boc.

Le dipeptide est obtenu est utilisé tel quel (après lavage avec 10 $cm^3$ d'eau afin d'éliminer le N-hydroxysuccinimide) dans l'étape de condensation suivante.

2ème étape : t-Boc-Phe-Met-AspOH
La déprotection du t-Boc dipeptide de l'étape 1 est réalisée par un traitement avec 0,5 ml d'acide trifluoroacétique pendant une heure à température ambiante.

La solution est ensuite évaporée et le résidu est repris dans 1,0 ml de triméthylcyanosilane.
Un équivalent de t-Boc-PheOSucc est ajouté.
Le couplage est réalisé comme précédemment décrit à la première étape pour le dipeptide.
Le tripeptide protégé par t-Boc est obtenu avec un rendement supérieur à 95 %.

3ème étape : t-Boc-Pro-Phe-Met-AspOH
Le protocole mentionné ci-dessus est répété en utilisant dans l'étape de condensation t-Boc-ProOSucc et on répète les opérations comme décrit pour la première étape.
Le N-hydroxysuccinimide formé est triméthylsilylé par l'addition de 0,5 ml de triméthylcyanosilane, il est ensuite évaporé sous vide.
Les résidus sont traités à l'eau puis sont lyophilisés.
Du tétrapeptide protégé pur est obtenu avec un rendement quantitatif.

Exemple 4 : Préparation de t-Boc-Thz-Phe-Met-AspOH

On opère comme décrit à l'exemple 3, les deux premières étapes sont identiques, mais lors de la troisième étape, on met en œuvre t-Boc-ThzOSucc à la place de t-Boc-ProOSucc.
Un rendement presque quantitatif de tétrapeptide est obtenu. Le produit est pur selon l'analyse de RMN.
Un mélange 40 : 60 d'isomères uréthane au niveau de l'agent de protection t-Boc est observé à 0 °C.

Exemple 5 : Préparation de diZ-Tyr-D-Ala-GlyOH

1ère étape : diZ-Tyr-D-AlaOH

449 mg de diZ-TyrOH (tyrosine hydratée protégée par un groupement de type dibenzyloxycarbonyle appelé diZ) sont dissous dans 5 ml de tétrahydrofurane.
On ajoute 0,096 ml de pyridine et 0,173 ml de triéthylamine, puis 0,144 ml de chlorure d'acide triméthylacétique (chlorure de pivaloyle, Piv-Cl) à — 10 °C afin d'obtenir un acide aminé protégé et activé.
Après une minute, 1 g d'un échangeur d'ions séché sous sa forme carboxylique libre (Amberlite IR C 50H) est ajouté afin de capturer tout excès de chlorure de pivaloyle.
Le mélange est agité durant 1 minute à — 10 °C.
107 mg de D-Ala sont dissous, en chauffant, dans 5 ml de triméthylcyanosilane ; on obtient l'acide aminé silylé qui est ajouté au mélange précédent.
Le mélange obtenu est porté à température ambiante et maintenu à cette température durant 30 minutes.
L'échangeur d'ions est filtré et le filtrat est lyophilisé.
On y ajoute 2 ml de méthanol et on évapore. On obtient diZ-Tyr-D-AlaOH qui est utilisé tel quel.

2ème étape : diZ-Tyr-D-Ala-FlyOH
Afin de l'activer, le dipeptide protégé obtenu lors de la première étape est dissous dans 0,096 ml de pyridine et 0,173 ml de triéthylamine puis avec 0,144 ml de chlorure de pivaloyle.
Après une minute, 1 g d'un échangeur d'ions séché sous sa forme carboxylique libre est ajouté et le mélange est agité une minute à — 10 °C.
90 mg de glycine sont dissous dans 5 ml de triméthylcyanosilane afin de réaliser la silylation. Ce produit silylé obtenu est ajouté au mélange qui est maintenu 30 minutes à température ambiante.
L'échangeur d'ions est filtré, le filtrat est lyophilisé et 2 ml de méthanol est ajouté.
Après évaporation, le tripeptide diZ-Tyr-Ala-GlyOH est rapidement recrystallisé dans un mélange éther éthylique : acétate d'éthyle, donnant le tripeptide pur par analyse RMN. Le rendement est de 68 %.

Exemple 6

Exemple 6.a : synthèse de la [$Met^5$]-encéphaline

La [$Met^5$]-encéphaline est un pentapeptide de formule Tyr-Gly-Gly-Phe-Met.

1ère étape : synthèse de t-Boc-Gly-Gly-Phe-MetOH
1,75 g (10 m.mol) de t-Boc-GlyOH (glycine hydratée protégée par le groupement t-Boc) est dissous avec 1,6 g (20 m.mol) de pyridine et avec 1,0 g (10 m.mol) de triéthylamine dans 20 ml de tétrahydrofurane séché.
La solution est refroidie à — 10 °C, on y ajoute 1,22 ml (10 m.mol) de chlorure de pivaloyle en agitant afin de réaliser l'étape d'activation.
Après une minute, on ajoute 750 mg (10 m.mol) de H-GlyOH qui sont préalablement dissous dans 6 ml de triméthylcyanosilane et 10 ml de n-hexane afin de silyler la glycine.
Le mélange est évaporé à une température supérieure à 60 °C.

On ajoute 20 ml de toluène et 1 ml de triméthylcyanosilane et le mélange est de nouveau évaporé sous vide afin d'éliminer le chlorure de pivaloyle en excès.

Le résidu est dissous dans 20 ml de méthanol puis est évaporé.

Le résidu est dissous dans 20 ml de toluène puis est évaporé.

Le dipeptide obtenu t-Boc-Gly-GlyOH est directement activé par le chlorure de pivaloyle puis couplé avec PheOH silylé comme décrit précédemment.

Le tripeptide obtenu t-Boc-Gly-Gly-PheOH est activé, puis couplé avec MetOH silylé dans les mêmes conditions précédemment décrites.

Le résidu final est dissous dans 100 ml d'acétate d'éthyle et 10 ml d'éthanol puis est lavé avec 50 ml d'acide citrique à 50 % dans l'eau.

La phase d'acétate d'éthyle est séchée sur du sulfate de magnésium puis est évaporée. Le résidu est recristallisé dans un mélange acétate d'éthyle : éther éthylique.

Le tétrapeptide t-Boc-Gly-Gly-Phe-MetOH est obtenu pur selon les critères de la RMN.

Le rendement est de 63 % (3,25 g).

2ème étape : synthèse de la [$Met^5$]-encéphaline

510 mg (1 m.mol) du tétrapeptide protégé par le groupement t-Boc, obtenu dans la première étape, sont dissous dans 2 ml d'acide trifluoroacétique (Tfa) qui contient 15 % en volume d'éthanethiol.

La solution est gardée en 2 heures à température ambiante, puis chauffée 15 minutes à 40 °C.

Après évaporation, on obtient la formation quantitative de Tfa-$H_3N^{\oplus}$-Gly-Gly-Phe-MetOH.

A ce produit est ajouté 415 mg (1,1 mol) de t-Boc-OH-Tyr-OSucc avec 2 ml de triméthylcyanosilane et 2 ml de tétrahydrofurane.

Le sel tétrapeptide est progressivement solubilisé, puis est passé 30 minutes aux ultrasons en agitant sous vide à 0 °C, une solution claire est ainsi obtenue. Après deux heures à température ambiante, la réaction est complète et le mélange est solidifié.

Le solide est dissous dans 5 ml de tétrahydrofurane.

L'excès de t-Boc-(OTMS)Tyr-OSucc est enlevé par l'addition de 1 g d'aminopropyl silicagel (5,40 μ, 3 meq · $NH_2$/g).

Après 30 minutes, le produit est filtré.

Le filtrat est traité avec 20 ml de méthanol.

Le produit est évaporé.

Le rendement obtenu lors de cette seconde étape est de 95 %.

Le pentapeptide protégé par t-Boc est recristallisé dans un mélange d'acétate d'éthyle : éther éthylique.

Le pentapeptide est obtenu pur selon les critères de RMN. Le rendement total est de 60 %.

Le pentapeptide [$Met^5$]-encéphaline est déprotégé par un traitement avec l'acide trifluoroacétique suivi d'une évaporation sous vide avec obtention du sel correspondant.

### Exemple 6.b : synthèse de la [$Leu^5$]-encéphaline

La [$Leu^5$]-encéphaline est un pentapeptide de formule Tyr-Gly-Gly-Phe-Leu.

La synthèse a lieu de la même manière que décrit dans l'exemple 6.a précédent, mais en remplaçant la méthionine par la leucine.

### Exemples 7 et 7R : Synthèse de t-Boc-Gly-Gly-Phe-MetOH

L'exemple 7 est conduit selon l'invention, l'exemple 7R est un exemple comparatif de l'exemple 7 qui utilise le triméthylchlorosilane en lieu et place du triméthylcyanosilane.

### Exemple 7 : Synthèse de t-Boc-Gly-Gly-Phe-MetOH

Dans un flacon de 500 ml sont mis 5 m.mol de t-Boc-Gly OH (glycine hydratée protégée par le groupement t-Boc) dissous dans 5 m.mol de triéthylamine, 5 m.mol de pyridine et 20 ml de tétrahydrofurane.

La solution est refroidie à — 15 °C et on y ajoute 5 m.mol de chlorure de pivaloyle en agitant afin de réaliser l'étape d'activation.

Après 2 minutes on ajoute 5,5 m.mol de glycine persilylée, obtenue en dissolvant H-Gly OH dans 3 ml de triméthylcyanosilane et 5 ml de n-hexane.

La solution est ramenée à température ambiante, puis évaporée sous pression réduite en vue de séparer les composants sylilés volatils.

La solution est traitée avec 30 ml de toluène, puis est évaporée à sec ; ceci est répété une fois avec de nouveau 30 ml de toluène.

Le résidu est dissous dans un mélange méthanol-toluène (1 : 1), puis est évaporé.

Le résidu est de nouveau traité deux fois avec 30 ml de toluène, puis est évaporé à sec en vue d'enlever toute trace d'excès de méthanol.

Le dipeptide obtenu t-Boc-Gly-Gly OH est directement activé par le chlorure de pivaloyle puis couplé avec Phe OH silylé comme décrit précédemment.

Le tripeptide obtenu t-Boc-Gly-Gly-Phe OH est activé, puis couplé avec MetOH silylé dans les mêmes conditions précédemment décrites.

Le résidu final évaporé contient t-Boc-Gly-Gly-Phe-MetOH.

Celui-ci est dissous dans 100 ml d'acétate d'éthyle et 20 ml de méthanol, puis est lavé successivement avec de l'eau et de l'acide phosphorique (pH environ 2).

Le résidu évaporé est recristallisé une première fois dans du dichlorométhane, une seconde fois dans un mélange acétate d'éthyle : éther éthylique.

Le tétrapeptide est obtenu pur selon les critères de la RMN.

Le rendement est de 63 %.

Exemple 7R — Exemple comparatif — Synthèse de t-Boc-Gly-Gly-Phe-MetOH

en utilisant comme agent de silylation le triméthylchlorosilane.

1ère étape : silylation de chaque acide aminé

a) Méthionine

A 5 m.mol de méthionine sous forme de zwitterion est ajouté 10 m.mol de triéthylamine dans 2 ml de triméthylchlorosilane. A ce mélange est ajouté 25 ml de trichlorométhane séché. Ce mélange est chauffé jusqu'à l'obtention de solutions claires.

On élimine le chlorhydrate de triméthylamine formé par filtration sous azote après précipitation au toluène séché.

Après filtration, la solution est réduite à environ 20 ml en volume et utilisée telle quelle.

La silylation de la méthionine est réalisée en 3 heures à température ambiante par cette méthode.

b) Glycine

La silylation de la glycine a lieu de la même manière que celle de la méthionine décrite dans l'exemple a) précédent ; mais cet acide aminé exige une étape de reflux.

c) Phénylalanine

La silylation de la phénylalanine a lieu de la même manière que celle de la méthionine décrite dans l'exemple a) précédent, mais cet acide aminé exige une étape de reflux.

2ème étape : activation

Dans un flacon de 500 ml sont mis 5 m.mol de t-Boc-GlyOH dissous dans 5 m.mol de triéthylamine, 5 m.mol de pyridine et 20 ml de tétrahydrofurane.

La solution est refroidie à —15 °C et on y ajoute 5 m.mol de chlorure de pivaloyle en agitant.

Après 2 minutes, on ajoute une solution de 5,5 m.mol de glycine persilylée obtenue lors de la première étape.

La solution est ramenée à température ambiante, puis évaporée sous pression réduite en vue de séparer les composants silylés volatils.

La solution est traitée avec 30 ml de toluène puis est évaporée à sec ; ceci est répété une fois avec de nouveau 30 ml de toluène.

Le résidu est dissous dans un mélange méthanol-toluène (1 : 1) ; puis est évaporé.

Le résidu est de nouveau traité deux fois avec 30 ml de toluène, puis est évaporé à sec en vue d'enlever toute trace d'excès de méthanol.

Le résidu évaporé est utilisé tel quel dans l'étape de couplage ultérieure.

Le même protocole est suivi avec la solution de phénylalanine persilylée en lieu et place de la glycine, puis avec la solution de méthionine persilylée, dans ce cas l'activation exige 10 minutes à —15 °C.

3ème étape : couplage

Le résidu final évaporé contient t-Boc-Gly-Gly-Phe-MetOH.

Celui-ci est dissous dans 100 ml d'acétate d'éthyle et 20 ml de méthanol, puis est lavé successivement avec de l'eau et de l'acide phosphorique (pH environ 2).

Le résidu évaporé est recristallisé une première fois dans du dichlorométhane, une seconde fois dans un mélange acétate d'éthyle : éther éthylique.

Le tétrapeptide est obtenu pur selon les critères de la RMN.

Le rendement est de 28 %.

La comparaison de l'exemple 7 et de l'exemple 7R montre la nette différence de rendement obtenu ; de plus, la réalisation de l'exemple 7R nécessite une succession d'opérations plus complexes (chauffage, filtration, évaporation) que l'exemple 7 ; tout particulièrement dans l'exemple 7R, la formation de triéthylamine-acide chlorhydrique (sels) est génante ; ce produit doit être éliminé par filtration après addition de toluène, ces opérations exigent des conditions de travail en atmosphère très sèche.

**Revendications**

1. Procédé pour la synthèse de peptides à partir :

a) d'acides aminés éventuellement associés et/ou substitués, qui sont activés, et

b) d'acides aminés éventuellement associés et/ou substitués, qui sont silylés à l'intervention de dérivés trialkylsilanes, caractérisé en ce qu'on utilise comme trialkylsilanes des trialkylcyanosilanes de formule générale (A)

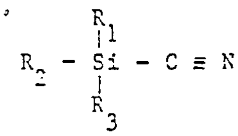

(A)

dans laquelle $R_1$, $R_2$, $R_3$ représentent indépendamment les uns des autres, des groupements alkyles, qui peuvent être identiques ou différents et qui contiennent de 1 à 3 atomes de carbone.

2. Procédé selon la revendication 1 caractérisé en ce que les trialkylcyanosilanes sont utilisés pour réaliser les liaisons peptidiques entre les acides aminés.

3. Procédé selon la revendication 1 ou 2, caractérisé en ce que les trois groupements alkyles $R_1$, $R_2$, $R_3$ contiennent de 1 à 2 atomes de carbone.

4. Procédé selon la revendication 3, caractérisé en ce que les trois groupements alkyles $R_1$, $R_2$, $R_3$ sont identiques.

5. Procédé selon la revendication 4, caractérisé en ce que le trialkylcyanosilane utilisé est le triméthylcyanosilane.

6. Procédé selon les revendications 1 à 4, caractérisé en ce que l'on met en œuvre de 20 à 0,01 ml de trialkylcyanosilane par m.mole d'acide aminé.

7. Procédé selon la revendication 5, caractérisé en ce que l'on met en œuvre de 5 à 0,1 ml de triméthylcyanosilane par m.mole d'acide aminé.

**Claims**

1. Process for synthesizing peptides from :

a) amino acids which may optionally be combined and/or substituted and which are activated, and

b) amino acids which may optionally be combined and/or substituted and which are silylated using trialkylsilane derivatives, characterized in that there are used, as trialkylsilanes, trialkylcyanosilanes of general formula (A)

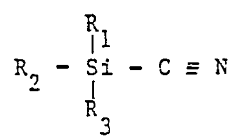

(A)

in which $R_1$, $R_2$, $R_3$ denote, independently of each other, alkyl groups which can be identical or different and which contain from 1 to 3 carbon atoms.

2. Process according to Claim 1, characterized in that the trialkylcyanosilanes are used to form peptide bonds between the amino acids.

3. Process according to Claim 1 or 2, characterized in that the three alkyl groups $R_1$, $R_2$ and $R_3$ contain 1 or 2 carbon atoms.

4. Process according to Claim 3, characterized in that the three alkyl groups $R_1$, $R_2$ and $R_3$ are identical.

5. Process according to Claim 4, characterized in that the trialkylcyanosilane used is trimethylcyanosilane.

6. Process according to Claims 1 to 4, characterized in that from 20 to 0.01 ml of trialkylcyanosilane are employed per mmol of amino acid.

7. Process according to Claim 5, characterized in that from 5 to 0.1 ml of trimethylcyanosilane are employed per mmol of amino acid.

**Patentansprüche**

1. Verfahren zur Synthese von Peptiden aus :

a) Aminosäuren, gegebenenfalls assoziiert und/oder substituiert, die aktiviert sind, und

b) Aminosäuren, gegebenenfalls assoziiert und/oder substituiert, die durch Einwirkung von Trialkylsilan-Derivaten silyliert sind, dadurch gekennzeichnet, daß man als Trialkylsilane Trialkylcyanosilane der allgemeinen Formel (A)

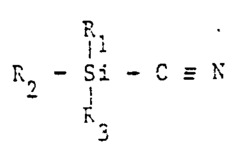

(A)

verwendet, worin $R_1$, $R_2$, $R_3$ unabhängig voneinander Alkylgruppen darstellen, die identisch oder unterschiedlich sein können und die von 1 bis 3 Kohlenstoffatome enthalten.

2. Verfahren nach Anspruch 1, dadurch gekennzeichnet, daß die Trialkylcyanosilane verwendet werden, um die Peptidbindungen zwischen den Aminosäuren zu verwirklichen.

3. Verfahren nach Anspruch 1 oder 2, dadurch gekennzeichnet, daß die drei Alkylgruppen $R_1$, $R_2$, $R_3$ von 1 bis 2 Kohlenstoffatome enthalten.

4. Verfahren nach Anspruch 3, dadurch gekennzeichnet, daß die drei Alkylgruppen $R_1$, $R_2$, $R_3$ identisch sind.

5. Verfahren nach Anspruch 4, dadurch gekennzeichnet, daß das verwendete Trialkylcyanosilan das Trimethylcyanosilan ist.

6. Verfahren nach den Ansprüchen 1 bis 4, dadurch gekennzeichnet, daß man 20 bis 0,01 ml Trialkylcyanosilan pro mmol Aminosäure einsetzt.

7. Verfahren nach Anspruch 5, dadurch gekennzeichnet, daß man von 5 bis 0,1 ml Trimethylcyanosilan pro mmol Aminosäure einsetzt.